# EUROPEAN PATENT SPECIFICATION

(11) **EP 3 458 154 B1**
(45) Date of publication and mention of the grant of the patent: **12.11.2025**
(21) Application number: 17748997.8
(22) Date of filing: 21.07.2017
(51) Int. Cl.: A61N 1/375, H01R 13/24

(54) **CONNECTOR ASSEMBLY WITH CONTACT RINGS COMPRISING BIASED BALL-SPRING CONTACTS**
STECKEREINHEIT MIT KONTAKTRINGEN MIT VORGESPANNTEN FEDERKONTAKTEN MIT JEWEILS EINER KUGEL
ENSEMBLE CONNECTEUR AVEC DES ANNEAUX DE CONTACT COMPRENANT DES CONTACTS À RESSORT PRÉCONTRAINT, POURVUS DE BILLES RESPECTIVES

(30) Priority: 29.07.2016 US 201662368610 P
(43) Date of publication of application: 27.03.2019
(73) Proprietor: Boston Scientific Neuromodulation Corporation, Valencia, CA 91355 (US)
(72) Inventor: NAGERI, Ranjan Krishna Mukhari, Valencia, CA 91354 (US)
(74) Representative: Vossius & Partner Patentanwälte Rechtsanwälte mbB
(86) International application number: PCT/US2017/043313
(87) International publication number: WO 2018/022455

(56) References cited:
- US-A- 5 968 082
- US-A1- 2002 095 079
- US-A1- 2002 128 692
- US-A1- 2013 288 500
- US-A1- 2014 148 885

## Description

### FIELD

The present invention is directed to the area of implantable electrical stimulation systems. The present invention is also directed to implantable electrical stimulation leads having biased ball-spring type contacts and connect assemblies, contact assemblies, and the electrical stimulation systems.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients. Stimulation of the brain, such as deep brain stimulation, can be used to treat a variety of diseases or disorders.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

US 2014/148885 A1 discloses a connector for an implantable medical device configured to couple to a proximal end of an inserted lead or lead extension. The connector includes a plurality of spherical connector contacts arranged in pockets. The connector contacts can retract to reduce the amount of force needed to insert the lead or lead extension into the connector. US 2002/095079 A1 discloses an electrical connector for multi-contact medical electrodes with plural-contact tails, including a tail-receiving member having a tail-receiving void defining an internal surface and an array of electrical conductors configured as spring-loaded ball plungers positioned along the internal surface.

### BRIEF SUMMARY

In one embodiment, a connector assembly includes an elongated connector housing having a first end, an opposing second end, and a longitudinal axis. The connector assembly further includes a port defined at the first end of the connector housing. The port is configured and arranged for receiving a proximal end of a lead or lead extension, wherein the proximal end of the lead or the lead extension includes a plurality of terminals electrically insulated from one another. The connector assembly further includes a lumen defined in the connector housing, and the lumen extends from the port along the longitudinal axis of the connector housing. Lastly, the connector assembly includes a plurality of contacts disposed in the elongated connector housing and electrically insulated from one another. At least one of the plurality of contacts is configured and arranged to couple to at least one of the plurality of terminals when the proximal end of the lead or lead extension is received within the lumen of the connector housing. Each of the plurality of contacts includes a contact ring and a plurality of ball-spring assemblies distributed around the contact ring. Each ball-spring assembly includes a housing coupled to the contract ring, a biasing member disposed within the ball-spring housing, and a conductive ball disposed at least partially within the ball-spring housing. The ball-spring housing defines an opening that is smaller than a diameter of the ball and the biasing member urges the ball towards the opening so that, absent a force countering the biasing member, a portion of the ball extends out of the opening in the ball-spring housing.

Another embodiment is an electrical contact for a lead assembly that includes a contact ring and a plurality of ball-spring assemblies distributed around the contact ring. Each ball-spring assembly includes a housing coupled to the contract ring, a biasing member disposed within the ball-spring housing, and a conductive ball disposed at least partially within the ball-spring housing. The ball-spring housing defines an opening that is smaller than a diameter of the ball and the biasing member urges the ball towards the opening so that, absent a force countering the biasing member, a portion of the ball extends out of the opening in the ball-spring housing.

In at least some embodiments, the ball-spring housing is a cylindrical body. In at least some embodiments, a fillet adjoins the ball-spring housing and an inner surface of the contact ring. In at least some embodiments, the ball is free to rotate within the ball-spring housing.

In at least some embodiments, each biasing member is a spring such as, but not limited to a helical compression spring or a conical compression spring. In at least some embodiments, the conductive ball is metallic. Optionally, the conductive ball is made from a conductive polymer or some other, non-metallic conductive material.

In at least some embodiments, the ball is free to translate within the housing. Additionally or alternatively, the biasing member is configured to move in a radial direction with respect to the longitudinal axis of the elongated connector housing. In at least some embodiments, the biasing member is further disposed between the ball and an inner surface of the contact ring.

Yet another embodiment is lead assembly that includes a lead or a lead extension having a proximal end and a distal end, wherein the proximal end of the lead or the lead extension includes a plurality of terminals electrically insulated from one another. The lead assembly also includes any of the connector assemblies described above.

A further embodiment is an electrical stimulating system includes any of the lead assemblies described above and a control module coupled to the lead assembly. The control module includes a housing, and an electronic subassembly disposed in the housing. In at least some embodiments, the connector assembly is part of the control module. In at least some embodiments, the lead assembly includes the lead and the electrical stimulation system further includes a lead extension coupleable to the control module and the lead, where the connector assembly is part of the lead extension.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system that includes a paddle body coupled to a control module via lead bodies, according to the invention;
FIG. 2 is a schematic view of another embodiment of an electrical stimulation system that includes a percutaneous lead body coupled to a control module via a lead body, according to the invention;
FIG. 3A is a schematic view of one embodiment of a plurality of connector assemblies disposed in the control module of FIG. 1, the connector assemblies configured and arranged to receive the proximal portions of the lead bodies of FIG. 1, according to the invention;
FIG. 3B is a schematic view of one embodiment of a connector assembly disposed in the control module of FIG. 2, the connector assembly configured and arranged to receive the proximal portion of one of the lead body of FIG. 2, according to the invention;
FIG. 3C is a schematic view of one embodiment of a proximal portion of the lead body of FIG. 2, a lead extension, and the control module of FIG. 2, the lead extension configured and arranged to couple the lead body to the control module, according to the invention;
FIG. 4A is a schematic, perspective view of a contact with ball-spring assemblies for a lead or a lead extension according to at least some embodiments of the present invention;
FIG. 4B is a close-up view, cross-sectional view of the contact of FIG. 4A;
FIG. 5 is a close-up view, cross-sectional view of the contact of FIG. 4A with a terminal concentrically disposed within ball-spring assemblies of the contact according to at least some embodiments of the present invention;
FIG. 6 is a schematic, perspective view of a contact with ball-spring assemblies showing an insertion or withdrawal direction for a lead or a lead extension according to at least some embodiments of the present invention;
FIG. 7 is a schematic, side-elevational view of a contact with ball-spring assemblies having conical springs according to at least some embodiments of the present invention;
FIG. 8 is a schematic, perspective view of a contact assembly with a plurality of ball-spring assemblies electrically insulated from each other according to at least some embodiments of the present invention; and
FIG. 9 is a schematic overview of one embodiment of components of a stimulation system, including an electronic subassembly disposed within a control module, according to the invention.

### DETAILED DESCRIPTION

The present invention is directed to the area of implantable electrical stimulation systems. The present invention is also directed to implantable electrical stimulation leads having biased ball-spring type contacts and connect assemblies, contact assemblies, and the electrical stimulation systems.

Suitable implantable electrical stimulation systems include, but are not limited to, a least one lead with one or more electrodes disposed along a distal end of the lead and one or more terminals disposed along the one or more proximal ends of the lead. Leads include, for example, percutaneous leads, paddle leads, and cuff leads. Examples of electrical stimulation systems with leads are found in, for example, U.S. Patents Nos. 6,181,969; 6,295,944; 6,391,985; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,831,742; 8,688,235; 6,175,710; 6,224,450; 6,271,094; 6,295,944; 6,364,278; and 6,391,985; U.S. Patent Applications Publication Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378;
2012/0046710; 2012/0071949; 2012/0165911; 2012/0197375; 2012/0203316; 2012/0203320; 2012/0203321; 2012/0316615; 2013/0105071; 2011/0005069; 2010/0268298; 2011/0130817; 2011/0130818; 2011/0078900; 2011/0238129; 2011/0313500; 2012/0016378; 2012/0046710; 2012/0165911; 2012/0197375; 2012/0203316; 2012/0203320; and 2012/0203321.

Examples of connectors, connector contacts and connector assemblies for electrical stimulation systems with leads are found in, for example, U.S. Patents Nos. 8,849,396; 7,244,150; 8,600,507; 8,897,876; 8,682,439; U.S. Patent Applications Publication Nos. 2012/0053646; 2014/0148885; 2015/0209575; 2016/0059019; and U.S. Patent Provisional Patent Application Nos. 62/193,472; 62/216,594; 62/259,463; and 62/278,667.

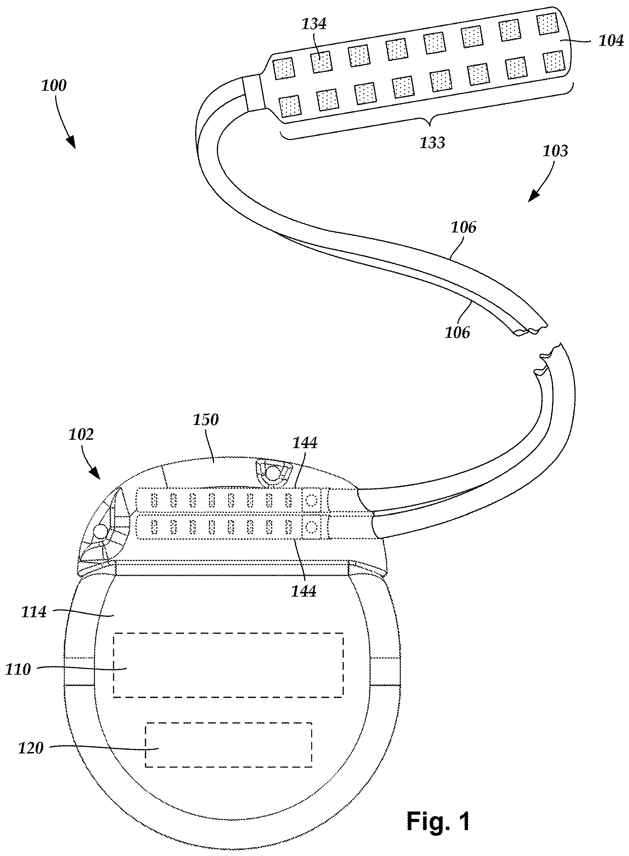

Figure 1 illustrates schematically one embodiment of an electrical stimulation system 100. The electrical stimulation system includes a control module (*e.g.,* a stimulator or pulse generator) 102 and a lead 103. The lead 103 including a paddle body 104 and one or more lead bodies 106 coupling the control module 102 to the paddle body 104. The paddle body 104 and the one or more lead bodies 106 form the lead 103. The paddle body 104 typically includes a plurality of electrodes 134 that form an array of electrodes 133. The control module 102 typically includes an electronic subassembly 110 and an optional power source 120 disposed in a sealed housing 114. In Figure 1, two lead bodies 106 are shown coupled to the control module 102.

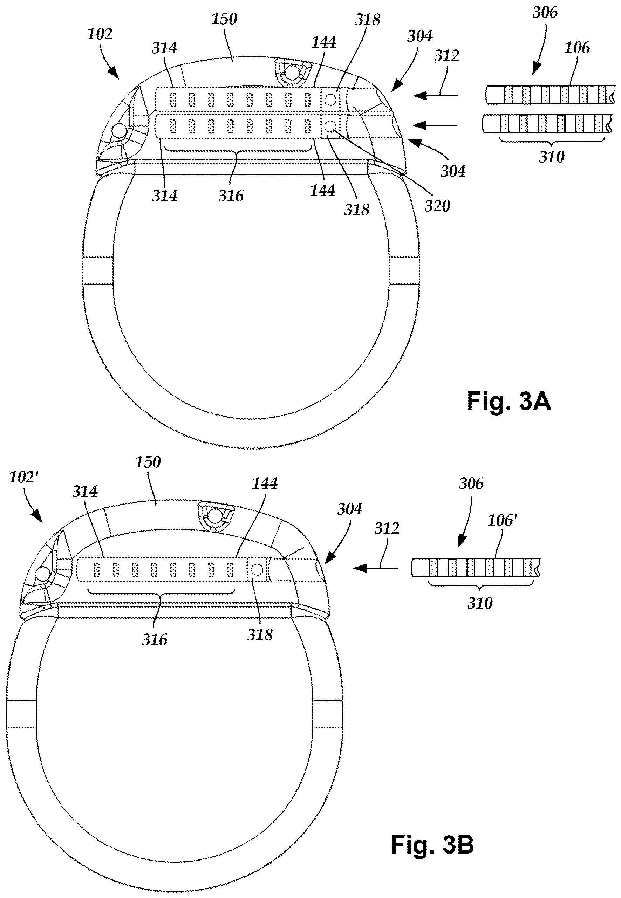

The control module 102 typically includes one or more connector assemblies 144 into which the proximal end of the one or more lead bodies 106 can be plugged to make an electrical connection via connector contacts (*e.g.,* 316 in Figure 3A) disposed in the connector assembly 144 and terminals (*e.g.,* 310 in Figure 3A) on each of the one or more lead bodies 106. The connector contacts are coupled to the electronic subassembly 110 and the terminals are coupled to the electrodes 134. In Figure 1, two connector assemblies 144 are shown.

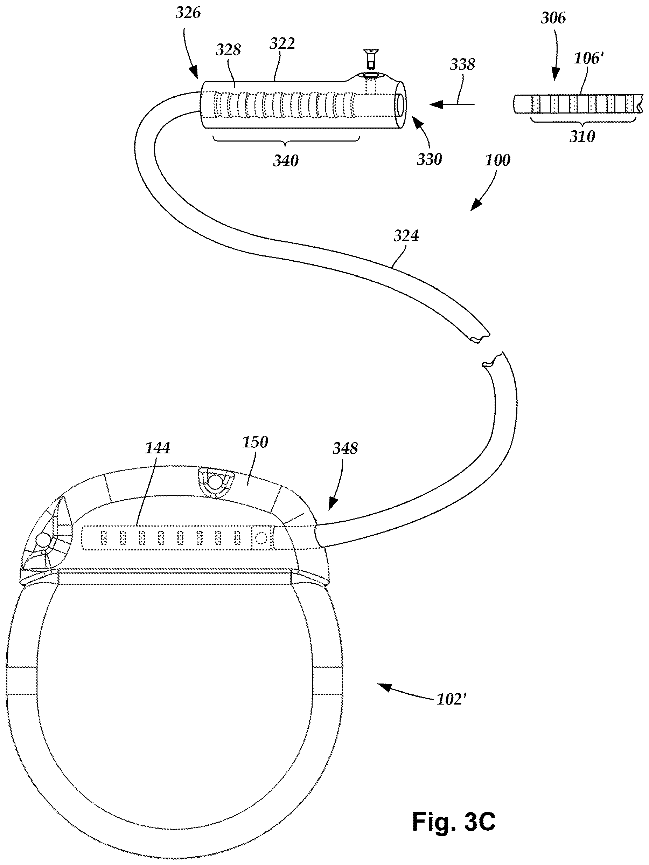

The one or more connector assemblies 144 may be disposed in a header 150. The header 150 provides a protective covering over the one or more connector assemblies 144. The header 150 may be formed using any suitable process including, for example, casting, molding (including injection molding), and the like. In addition, one or more lead extensions 324 (see Figure 3C) can be disposed between the one or more lead bodies 106 and the control module 102 to extend the distance between the one or more lead bodies 106 and the control module 102.

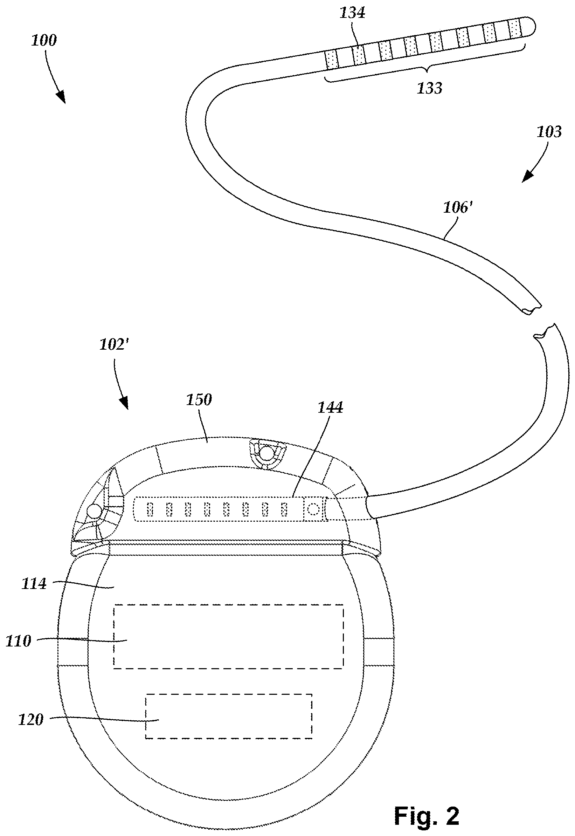

It will be understood that the electrical stimulation system can include more, fewer, or different components and can have a variety of different configurations including those configurations disclosed in the electrical stimulation system references cited herein. For example, instead of a paddle body 104, the electrodes 134 can be disposed in an array at or near the distal end of a lead body 106' forming a percutaneous lead 103, as illustrated in Figure 2. The percutaneous lead may be isodiametric along the length of the lead body 106". The lead body 106' can be coupled with a control module 102' with a single connector assembly 144.

The electrical stimulation system or components of the electrical stimulation system, including one or more of the lead bodies 106, the control module 102, and, in the case of a paddle lead, the paddle body 104, are typically implanted into the body of a patient. The electrical stimulation system can be used for a variety of applications including, but not limited to, spinal cord stimulation, brain stimulation, neural stimulation, muscle activation via stimulation of nerves innervating muscle, and the like.

The electrodes 134 can be formed using any conductive, biocompatible material. Examples of suitable materials include metals, alloys, conductive polymers, conductive carbon, and the like, as well as combinations thereof. In at least some embodiments, one or more of the electrodes 134 are formed from one or more of: platinum, platinum iridium, palladium, titanium, or rhenium.

The number of electrodes 134 in the array of electrodes 133 may vary. For example, there can be two, three, four, five, six, seven, eight, nine, ten, eleven, twelve, thirteen, fourteen, fifteen, sixteen, or more electrodes 134. As will be recognized, other numbers of electrodes 134 may also be used. In Figure 1, sixteen electrodes 134 are shown. The electrodes 134 can be formed in any suitable shape including, for example, round, oval, triangular, rectangular, pentagonal, hexagonal, heptagonal, octagonal, or the like.

The electrodes of the paddle body 104 or one or more lead bodies 106 are typically disposed in, or separated by, a non-conductive, biocompatible material including, for example, silicone, polyurethane, and the like or combinations thereof. The paddle body 104 and one or more lead bodies 106 may be formed in the desired shape by any process including, for example, molding (including injection molding), casting, and the like. Electrodes and connecting wires can be disposed onto or within a paddle body either prior to or subsequent to a molding or casting process. The non-conductive material typically extends from the distal end of the lead 103 to the proximal end of each of the one or more lead bodies 106. The non-conductive, biocompatible material of the paddle body 104 and the one or more lead bodies 106 may be the same or different. The paddle body 104 and the one or more lead bodies 106 may be a unitary structure or can be formed as two separate structures that are permanently or detachably coupled together.

Terminals (*e.g*., 310 in Figure 3A) are typically disposed at the proximal end of the one or more lead bodies 106 for connection to corresponding conductive contacts (*e.g.,* 316 in Figure 3A) in connector assemblies (*e.g.,* 144 in Figure 1) disposed on, for example, the control module 102 (or to other devices, such as conductive contacts on a lead extension, an operating room cable, a splitter, an adaptor, or the like).

Conductive wires (not shown) extend from the terminals (*e.g*., 310 in Figure 3A) to the electrodes 134. Typically, one or more electrodes 134 are electrically coupled to a terminal (*e.g.,* 310 in Figure 3A). In some embodiments, each terminal (*e.g.,* 310 in Figure 3A) is only coupled to one electrode 134.

The conductive wires may be embedded in the non-conductive material of the lead or can be disposed in one or more lumens (not shown) extending along the lead. In some embodiments, there is an individual lumen for each conductive wire. In other embodiments, two or more conductive wires may extend through a lumen. There may also be one or more lumens (not shown) that open at, or near, the proximal end of the lead, for example, for inserting a stylet rod to facilitate placement of the lead within a body of a patient. Additionally, there may also be one or more lumens (not shown) that open at, or near, the distal end of the lead, for example, for infusion of drugs or medication into the site of implantation of the paddle body 104. The one or more lumens may, optionally, be flushed continually, or on a regular basis, with saline, epidural fluid, or the like. The one or more lumens can be permanently or removably sealable at the distal end.

As discussed above, the one or more lead bodies 106 may be coupled to the one or more connector assemblies 144 disposed on the control module 102. The control module 102 can include any suitable number of connector assemblies 144 including, for example, two three, four, five, six, seven, eight, or more connector assemblies 144. It will be understood that other numbers of connector assemblies 144 may be used instead. In Figure 1, each of the two lead bodies 106 includes eight terminals that are shown coupled with eight conductive contacts disposed in a different one of two different connector assemblies 144.

Figure 3A is a schematic side view of one embodiment of a plurality of connector assemblies 144 disposed on the control module 102. In at least some embodiments, the control module 102 includes two connector assemblies 144. In at least some embodiments, the control module 102 includes four connector assemblies 144. In Figure 3A, proximal ends 306 of the plurality of lead bodies 106 are shown configured and arranged for insertion to the control module 102. Figure 3B is a schematic side view of one embodiment of a single connector assembly 144 disposed on the control module 102'. In Figure 3B, the proximal end 306 of the single lead body 106' is shown configured and arranged for insertion to the control module 102'.

In Figures 3A and 3B, the one or more connector assemblies 144 are disposed in the header 150. In at least some embodiments, the header 150 defines one or more ports 304 into which the proximal end(s) 306 of the one or more lead bodies 106/106' with terminals 310 can be inserted, as shown by directional arrows 312, in order to gain access to the connector contacts disposed in the one or more connector assemblies 144.

The one or more connector assemblies 144 each include a connector housing 314 and a plurality of connector contacts 316 disposed therein. Typically, the connector housing 314 defines a port (not shown) that provides access to the plurality of connector contacts 316. In at least some embodiments, one or more of the connector assemblies 144 further includes a retaining element 318 configured and arranged to fasten the corresponding lead body 106/106' to the connector assembly 144 when the lead body 106/106' is inserted into the connector assembly 144 to prevent undesired detachment of the lead body 106/106' from the connector assembly 144. For example, the retaining element 318 may include an aperture 320 through which a fastener (*e.g.,* a set screw, pin, or the like) may be inserted and secured against an inserted lead body 106/106'.

When the one or more lead bodies 106/106' are inserted into the one or more ports 304, the connector contacts 316 can be aligned with the terminals 310 disposed on the one or more lead bodies 106/106' to electrically couple the control module 102 to the electrodes (134 of Figure 1) disposed at a distal end of the one or more lead bodies 106. Examples of connector assemblies in control modules are found in, for example, U.S. Patents Nos. 7,244,150 and 8,224,450.

In at least some embodiments, the electrical stimulation system includes one or more lead extensions. The one or more lead bodies 106/106' can be coupled to one or more lead extensions which, in turn, are coupled to the control module 102/102'. In Figure 3C, a lead extension connector assembly 322 is disposed on a lead extension 324. The lead extension connector assembly 322 is shown disposed at a distal end 326 of the lead extension 324. The lead extension connector assembly 322 includes a contact housing 328. The contact housing 328 defines at least one port 330 into which a proximal end 306 of the lead body 106' with terminals 310 can be inserted, as shown by directional arrow 338. The lead extension connector assembly 322 also includes a plurality of connector contacts 340. When the lead body 106' is inserted into the port 330, the connector contacts 340 disposed in the contact housing 328 can be aligned with the terminals 310 on the lead body 106 to electrically couple the lead extension 324 to the electrodes (134 of Figure 1) disposed at a distal end (not shown) of the lead body 106'.

The proximal end of a lead extension can be similarly configured and arranged as a proximal end of a lead body. The lead extension 324 may include a plurality of conductive wires (not shown) that electrically couple the connector contacts 340 to terminal on a proximal end 348 of the lead extension 324. The conductive wires disposed in the lead extension 324 can be electrically coupled to a plurality of terminals (not shown) disposed on the proximal end 348 of the lead extension 324. In at least some embodiments, the proximal end 348 of the lead extension 324 is configured and arranged for insertion into a lead extension connector assembly disposed in another lead extension. In other embodiments (as shown in Figure 3C), the proximal end 348 of the lead extension 324 is configured and arranged for insertion into the connector assembly 144 disposed on the control module 102'.

It will be understood that the control modules 102/102' can receive either lead bodies 106/106' or lead extensions 324. It will also be understood that the electrical stimulation system 100 can include a plurality of lead extensions 224. For example, each of the lead bodies 106 shown in Figures 1 and 3A can, alternatively, be coupled to a different lead extension 224 which, in turn, are each coupled to different ports of a two-port control module, such as the control module 102 of Figures 1 and 3A.

In at least some conventional electrical stimulation systems, coupling a neuromodulation lead to a lead extension or coupling a lead or lead extension to an implantable pulse generator (IPG) header is accomplished using canted coil spring contacts within the IPG header. An alternative contact assembly, described below, includes a plurality of contact rings with ball-spring assemblies. Such an arrangement may reduce an insertion force of a proximal end portion of a lead or lead extension during insertion into or withdrawal from a mating component such as an IPG header, a connector on a lead extension or any other connector. Biasing members, which may take the form of springs, urge spherical balls into contact with the lead or lead extension to keep the lead or lead extension concentrically aligned with the contact, reduce the frictional forces on the lead or lead extension (*e.g*., reduce the drag), and thus lower or reduce the insertion or withdrawal force of the lead or lead extension vis-à-vis the mating component.

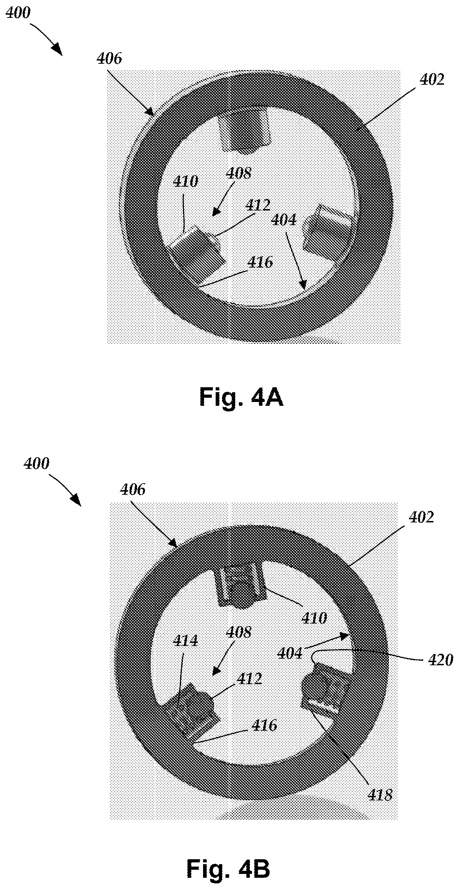

Figures 4A and 4B show schematic, perspective views of a contact 400 having a contact ring 402 with an inner surface 404 and an outer surface 406. A plurality of ball-spring assemblies 408 project or extend from the inner surface 404. Each ball-spring assembly 408 includes a ball-spring housing 410, a ball 412 and a biasing member 414 (Fig. 4B). The ball-spring housing 410 can be attached to the inner surface 404 of the contact ring 402 by a variety of techniques such as, but not limited to, welding or bonding. In at least one embodiment, the ball-spring housing 410 can be attached to the inner surface 404 of the contact ring 402 by a metal molding technique. In the illustrated embodiment, the ball-spring housing 410 is welded to the inner surface 404 of the contact ring 402, which generates a fillet 416. The ball-spring housing 410 may take the form of a cylindrical body, but it is appreciated that other shapes are possible. The ball 414 may take the form of a spherically shaped ball that made be made from a conductive material such as, but not limited to, a metallic or other conductive material like a conductive polymer. It is appreciated that the ball, the biasing member and the contact ring are made from one or more conductive materials to form a conductive path with the lead terminal.

In a preferred embodiment, at least three ball-spring assemblies 408 advantageously provide at least three points of contact for a lead or a lead extension (hereinafter lead) during insertion or withdrawal of the lead from the contact 400. The three ball-spring assemblies 408 permit the lead to be concentrically located relative to the contact ring 402. Additionally or alternatively, the frictional forces on the lead during insertion or withdrawal depend, at least in part, on the spring constant (*e.g*., spring force) of the biasing member. Using at least three ball-spring assemblies 408 supports the lead concentrically during insertion and withdrawal. Alternatively, more than three ball-spring assemblies 408 may be disposed on the contact ring 402.

Referring to Figure 4B, the ball-spring housing 408 includes a wall 418 and a stop 420. The wall 418 extending from the inner surface 404 of the contact ring 402 and is preferably a continuous, cylindrical member. The stop 420 is integrally formed with or coupled to the wall 418. The stop 420 defines an opening (located where the ball 412 is seated in Fig. 4B). The opening, in turn, is smaller than a diameter of the ball 412, which in turn prevents the ball from being pushed out of or otherwise falling out of the ball-spring housing 410. In at least some embodiments, the bore sized of the ball-spring housing 410 in cooperation with the narrow opening permits the ball 412 to rotate and move freely within the ball-spring housing 410, wherein the movement of the ball includes translation of the ball 412 in a radial direction relative to a longitudinal axis of the contact ring 402 or relative to the longitudinal axis of the elongated connector housing, which is coincident to the longitudinal axis of the contact ring 402. However, the radial translation of the ball 412 is constrained by cooperation of the stop 420 and the biasing member 414.

In at least some embodiment, the biasing member 414 takes the form of a helical compression spring. As noted above, the spring constant may be adjustable or modified prior to assembly, which in turn would require either more or less force to move the ball 412 outward toward the inner surface 404 of the contact ring 402 during insertion, withdrawal, or both of the lead or lead extension. The biasing member 414 is disposed within the ball-spring housing 410, and more specifically disposed between the ball 412 and the inner surface 404 of the contact ring 402.

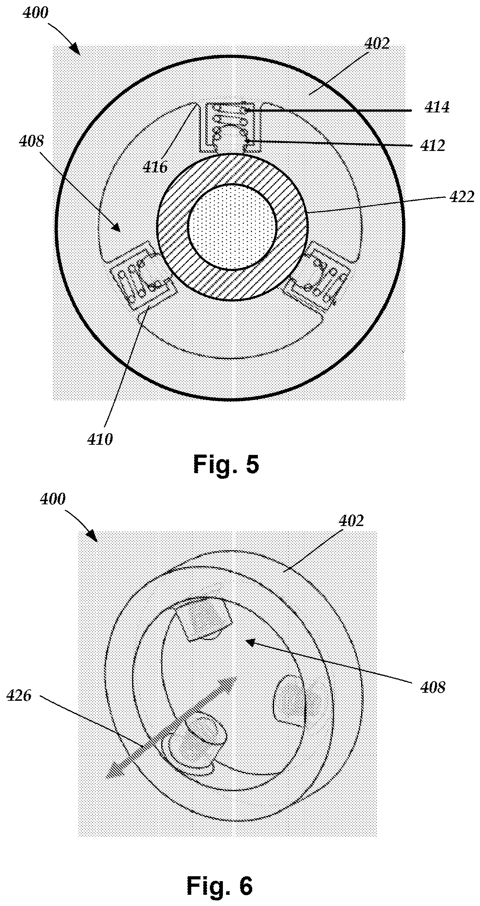

Figure 5 shows a schematic, cross-sectional view of the contact 400 with a terminal 422 concentrically disposed within the contact 400. The terminal 422 may take the form of terminals 310 in Fig. 3C.

Figure 6 shows a schematic, perspective view of the contact 400. The arrow 426 indicates the insertion or withdrawal direction of the lead or lead extension vis-à-vis the contact 400. Even if the lead or lead extension is not directly aligned with the contact 400, the ball-spring assemblies 408 may operate to concentrically align or position the lead or lead extension during insertion or withdrawal.

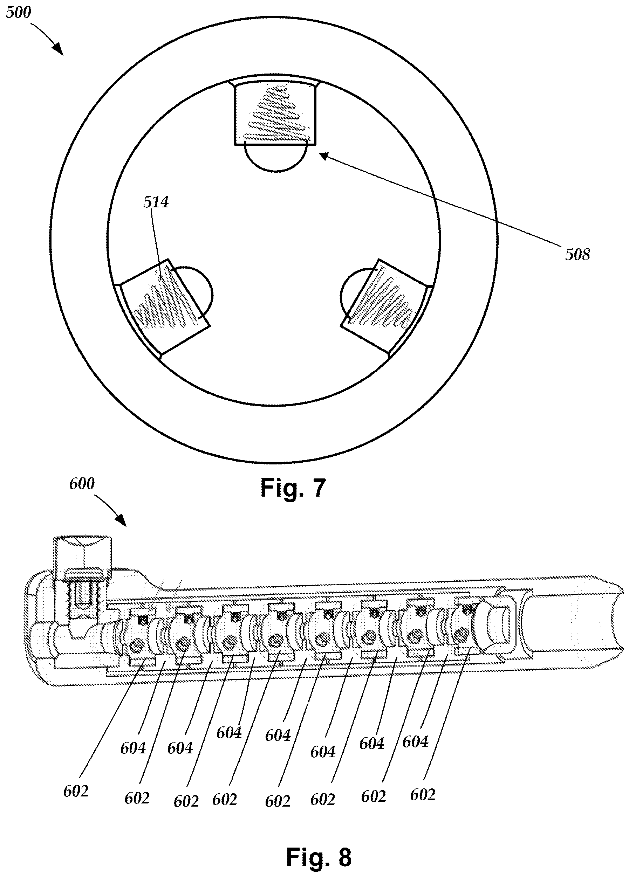

Figure 7 shows a schematic, cross-sectional view of another contact 500 that includes the same features, aspects and components as the contact 400 except that the biasing members 514 disposed within the ball-spring assemblies 508 take the form of conical springs, as compared to helical springs. In at least some embodiments, the biasing members 514 may take other types of springs such as, but not limited to, hourglass and barrel-shaped springs or various types of flat springs. Any of the springs described herein may advantageously provide a low solid height with lateral stability or resistance to surging. In at least some embodiments, conical springs can be selected or designed so that each coil nests wholly or partly into an adjacent coil. The solid height can be as low as one wire diameter. Additionally or alternatively, the spring constant or spring rate for conical springs generally increases with deflection because the number of active coils decreases progressively as the spring approaches solid. Although by varying the pitch, conical springs can be designed to have a uniform rate.

In at least some other embodiments, the biasing member may take the form of a compressible or viscous fluid or some other type of device capable of urging and maintaining the ball radially inward relative to the contact ring.

Figure 8 shows a schematic, perspective view of a connector assembly 600. In at least some embodiments, the connector assembly 600 includes a plurality of contacts 602 that are electrically insulated from one another with a silicon material 604. By way of example, the contacts may be disposed in, or separated by, a non-conductive, biocompatible material such as, for example, silicone, polyurethane, polyetheretherketone ("PEEK"), epoxy, and the like or combinations thereof. The contacts themselves can be formed using any conductive, biocompatible material. Examples of suitable materials include metals, alloys, conductive polymers, conductive carbon, and the like, as well as combinations thereof. In at least some embodiments, one or more of the contacts are formed from one or more of: platinum, platinum iridium, palladium, palladium rhodium, or titanium.

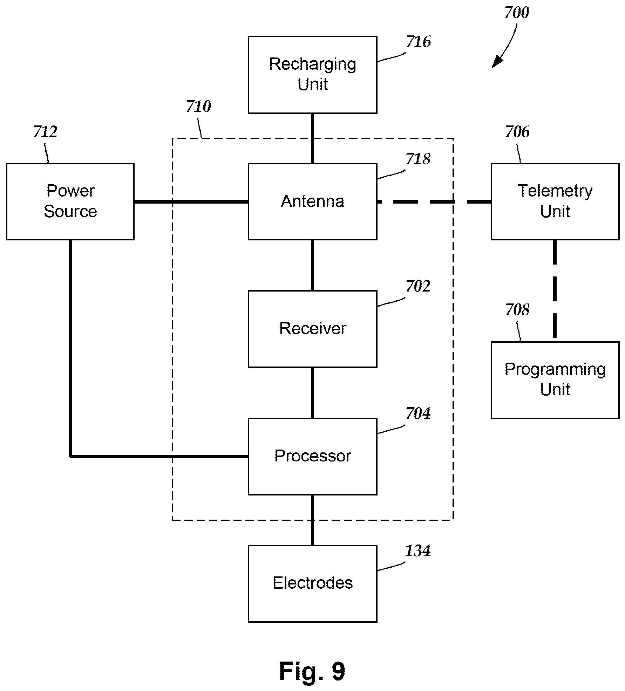

Figure 9 is a schematic overview of one embodiment of components of an electrical stimulation system 800 including an electronic subassembly 810 disposed within a control module. It will be understood that the electrical stimulation system can include more, fewer, or different components and can have a variety of different configurations including those configurations disclosed in the stimulator references cited herein.

Some of the components (for example, a power source 812, an antenna 818, a receiver 802, and a processor 804) of the electrical stimulation system can be positioned on one or more circuit boards or similar carriers within a sealed housing of an implantable pulse generator, if desired. Any power source 812 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193.

As another alternative, power can be supplied by an external power source through inductive coupling via the optional antenna 818 or a secondary antenna. The external power source can be in a device that is mounted on the skin of the user or in a unit that is provided near the user on a permanent or periodic basis.

If the power source 812 is a rechargeable battery, the battery may be recharged using the optional antenna 818, if desired. Power can be provided to the battery for recharging by inductively coupling the battery through the antenna to a recharging unit 816 external to the user. Examples of such arrangements can be found in the references identified above.

In one embodiment, electrical current is emitted by the electrodes 134 on the paddle or lead body to stimulate nerve fibers, muscle fibers, or other body tissues near the electrical stimulation system. The processor 804 is generally included to control the timing and electrical characteristics of the electrical stimulation system. For example, the processor 804 can, if desired, control one or more of the timing, frequency, strength, duration, and waveform of the pulses. In addition, the processor 804 can select which electrodes can be used to provide stimulation, if desired. In some embodiments, the processor 804 selects which electrode(s) are cathodes and which electrode(s) are anodes. In some embodiments, the processor 804 is used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 808 that, for example, allows modification of pulse characteristics. In the illustrated embodiment, the processor 804 is coupled to a receiver 802 which, in turn, is coupled to the optional antenna 818. This allows the processor 804 to receive instructions from an external source to, for example, direct the pulse characteristics and the selection of electrodes, if desired.

In one embodiment, the antenna 818 is capable of receiving signals (*e.g.,* RF signals) from an external telemetry unit 806 which is programmed by the programming unit 808. The programming unit 808 can be external to, or part of, the telemetry unit 806. The telemetry unit 806 can be a device that is worn on the skin of the user or can be carried by the user and can have a form similar to a pager, cellular phone, or remote control, if desired. As another alternative, the telemetry unit 806 may not be worn or carried by the user but may only be available at a home station or at a clinician's office. The programming unit 808 can be any unit that can provide information to the telemetry unit 806 for transmission to the electrical stimulation system 800. The programming unit 808 can be part of the telemetry unit 806 or can provide signals or information to the telemetry unit 806 via a wireless or wired connection. One example of a suitable programming unit is a computer operated by the user or clinician to send signals to the telemetry unit 806.

The signals sent to the processor 804 via the antenna 818 and the receiver 802 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 800 to cease operation, to start operation, to start charging the battery, or to stop charging the battery. In other embodiments, the stimulation system does not include the antenna 818 or receiver 802 and the processor 804 operates as programmed.

Optionally, the electrical stimulation system 800 may include a transmitter (not shown) coupled to the processor 804 and the antenna 818 for transmitting signals back to the telemetry unit 806 or another unit capable of receiving the signals. For example, the electrical stimulation system 800 may transmit signals indicating whether the electrical stimulation system 800 is operating properly or not or indicating when the battery needs to be charged or the level of charge remaining in the battery. The processor 804 may also be capable of transmitting information about the pulse characteristics so that a user or clinician can determine or verify the characteristics.

The above specification provides a description of the structure, manufacture, and use of the invention. Since many embodiments of the invention can be made without departing from the scope of the invention as defined by the appended claims.

## Claims

1. A connector assembly (144; 322) comprising:
an elongated connector housing (314) having a first end, an opposing second end, and a longitudinal axis;
a port (304; 330) defined at the first end of the connector housing (314; 324), the port (304; 330) configured and arranged for receiving a proximal end (306; 348) of a lead or lead extension (103; 324), wherein the proximal end (306; 348) of the lead or the lead extension (103; 324) includes a plurality of terminals (310) electrically insulated from one another;
a lumen defined in the connector housing (314; 324), the lumen extending from the port (304; 330) along the longitudinal axis of the connector housing (314; 324); and
a plurality of contacts (316; 400; 500; 602) disposed in the elongated connector housing (314; 324) and electrically insulated from one another, at least one of the plurality of contacts (316; 400) configured and arranged to couple to at least one of the plurality of terminals (310) when the proximal end of the lead or lead extension (103; 324) is received within the lumen of the connector housing (314; 324);
wherein each of the plurality of contacts (316; 400; 500) comprises a contact ring (402) and a plurality of ball-spring assemblies (408; 508) distributed around the contact ring (402), each ball-spring assembly (408; 508) comprising a housing (410) coupled to the contact ring (402), a biasing member (414; 514) disposed within the ball-spring housing (410), and a conductive ball (412) disposed at least partially within the ball-spring housing (410), wherein the ball-spring housing (410) defines an opening that is smaller than a diameter of the ball (412) and the biasing member (414; 514) urges the ball (412) towards the opening so that, absent a force countering the biasing member (414; 514), a portion of the ball (412) extends out of the opening in the ball-spring housing (410).

2. The connector assembly (144; 322) of claim 1, wherein the ball-spring housing (410) is a cylindrical body.

3. The connector assembly (144; 322) of any one of claims 1 or 2, further comprising a fillet (416) adjoining the ball-spring housing (410) and an inner surface (404) of the contact ring (402).

4. The connector assembly (144; 322) of any one of claims 1-3, wherein the ball (412) is free to rotate within the ball-spring housing (410).

5. The connector assembly (144; 322) of any one of claims 1-4, wherein the biasing member (414; 514) is either a helical or conical compression spring.

6. The connector assembly (144; 322) of any one of claims 1-5, wherein the ball (412) is free to translate within the housing (410).

7. The connector assembly of claim 6, wherein the biasing member (414; 514) is configured to move in a radial direction with respect to the longitudinal axis of the elongated connector housing (314; 324).

8. The connector assembly (144; 322) of any one of claims 1-7, wherein the biasing member (414; 514) is further disposed between the ball (412) and an inner surface (404) of the contact ring (402).

9. A lead assembly comprising:
a lead or a lead extension (103; 324) having a proximal end (306; 348) and a distal end (326), wherein the proximal end (306; 348) of the lead or the lead extension (103; 324) includes a plurality of terminals (310) electrically insulated from one another; and
the connector assembly (144; 322) of any one of claims 1-8.

10. An electrical stimulating system (100; 800) comprising:
the lead assembly of claim 9; and
a control module (102) coupled to the lead assembly, the control module comprising
a housing (114), and
an electronic subassembly (110; 810) disposed in the housing (114).

11. The electrical stimulation system (100; 800) of claim 10, wherein the connector assembly (144; 322) is part of the control module (102).

12. The electrical stimulation system (100; 800) of claim 10, wherein the lead assembly comprises the lead extension (324) and the electrical stimulation system (100; 800) further comprises a lead (103) coupleable to the control module (102) and the lead extension (324), wherein the connector assembly (322) is part of the lead extension (324).

13. An electrical contact (400; 500) for a lead assembly, the electrical contact (400; 500) comprising:
a contact ring (402); and
a plurality of ball-spring assemblies (408; 508) distributed around the contact ring (402), each ball-spring assembly (408; 508) comprising a housing (410) coupled to the contact ring (402), a biasing member (414; 514) disposed within the ball-spring housing (410), and a conductive ball (412) disposed at least partially within the ball-spring housing (410), wherein the ball-spring housing (410) defines an opening that is smaller than a diameter of the ball (412) and the biasing member (414; 514) urges the ball (412) towards the opening so that, absent a force countering the biasing member (414; 514), a portion of the ball (412) extends out of the opening in the ball-spring housing (410).

14. The electrical contact (400; 500) of claim 13, wherein the ball (412) is free to rotate within the ball-spring housing (410).

15. The electrical contact (400; 500) of any one of claims 13 or 14, wherein the biasing member (414; 514) is a helical or conical compression spring.

## Patentansprüche

1. Verbinderanordnung (144; 322), aufweisend:
ein längliches Verbindergehäuse (314) mit einem ersten Ende, einem gegenüberliegenden zweiten Ende und einer Längsachse;
einen Anschluss (304; 330), der am ersten Ende des Verbindergehäuses (314; 324) definiert ist, wobei der Anschluss (304; 330) zum Aufnehmen eines proximalen Endes (306; 348) einer Leitung oder Leitungsverlängerung (103; 324) konfiguriert und angeordnet ist, wobei das proximale Ende (306; 348) der Leitung oder der Leitungsverlängerung (103; 324) mehrere Anschlüsse (310) aufweist, die elektrisch voneinander isoliert sind;
ein Lumen, das im Verbindergehäuse (314; 324) definiert ist, wobei sich das Lumen vom Anschluss (304; 330) entlang der Längsachse des Verbindergehäuses (314; 324) erstreckt; und
mehrere Kontakte (316; 400; 500; 602), die im länglichen Verbindergehäuse (314; 324) angeordnet und voneinander elektrisch isoliert sind, wobei mindestens einer der mehreren Kontakte (316; 400) dazu eingerichtet und angeordnet ist, mit mindestens einem der mehreren Anschlüsse (310) zu koppeln, wenn das proximale Ende der Leitung oder Leitungsverlängerung (103; 324) im Lumen des Verbindergehäuses (314; 324) aufgenommen ist;
wobei jeder der mehreren Kontakte (316; 400; 500) einen Kontaktring (402) und mehrere um den Kontaktring (402) verteilte Kugel-Feder-Anordnungen (408; 508) aufweist, wobei jede Kugel-Feder-Anordnung (408; 508) aufweist: ein Gehäuse (410), das mit dem Kontaktring (402) gekoppelt ist, ein Vorspannteil (414; 514), das im Kugel-Feder-Gehäuse (410) angeordnet ist, und eine leitende Kugel (412), die mindestens teilweise im Kugel-Feder-Gehäuse (410) angeordnet ist, wobei das Kugel-Feder-Gehäuse (410) eine Öffnung definiert, die kleiner ist als ein Durchmesser der Kugel (412), und das Vorspannteil (414; 514) die Kugel (412) hin zur Öffnung drückt, so dass bei Fehlen einer dem Vorspannteil (414; 514) entgegenwirkenden Kraft sich ein Abschnitt der Kugel (412) aus der Öffnung im Kugel-Feder-Gehäuse (410) erstreckt.

2. Verbinderanordnung (144; 322) nach Anspruch 1, wobei das Kugel-Feder-Gehäuse (410) ein zylindrischer Körper ist.

3. Verbinderanordnung (144; 322) nach Anspruch 1 oder 2, ferner mit einer Ausrundung (416), die an das Kugel-Feder-Gehäuse (410) und eine Innenfläche (404) des Kontaktrings (402) angrenzt.

4. Verbinderanordnung (144; 322) nach einem der Ansprüche 1 bis 3, wobei die Kugel (412) im Kugel-Feder-Gehäuse (410) frei drehen kann.

5. Verbinderanordnung (144; 322) nach einem der Ansprüche 1 bis 4, wobei das Vorspannteil (414; 514) eine Spiral- oder Kegeldruckfeder ist.

6. Verbinderanordnung (144; 322) nach einem der Ansprüche 1 bis 5, wobei die Kugel (412) im Gehäuse (410) frei linear verschiebbar ist.

7. Verbinderanordnung nach Anspruch 6, wobei das Vorspannteil (414; 514) dazu eingerichtet ist, sich in einer Radialrichtung im Hinblick auf die Längsachse des länglichen Verbindergehäuses (314; 324) zu bewegen.

8. Verbinderanordnung (144; 322) nach einem der Ansprüche 1 bis 7, wobei das Vorspannteil (414; 514) ferner zwischen der Kugel (412) und einer Innenfläche (404) des Kontaktrings (402) angeordnet ist.

9. Leitungsanordnung, die aufweist:
eine Leitung oder eine Leitungsverlängerung (103; 324) mit einem proximalen Ende (306; 348) und einem distalen Ende (326), wobei das proximale Ende (306; 348) der Leitung oder der Leitungsverlängerung (103; 324) mehrere Anschlüsse (310) aufweist, die elektrisch voneinander isoliert sind; und
die Verbinderanordnung (144; 322) nach einem der Ansprüche 1 bis 8.

10. Elektrisches Stimulationssystem (100; 800), das aufweist;
die Leitungsanordnung nach Anspruch 9; und
ein Steuermodul (102), das mit der Leitungsanordnung gekoppelt ist, wobei das Steuermodul aufweist:
ein Gehäuse (114) und
eine elektronische Teilanordnung (110; 810), die im Gehäuse (114) angeordnet ist.

11. Elektrisches Stimulationssystem (100; 800) nach Anspruch 10, wobei die Verbinderanordnung (144; 322) Teil des Steuermoduls (102) ist.

12. Elektrisches Stimulationssystem (100; 800) nach Anspruch 10, wobei die Leitungsanordnung die Leitungsverlängerung (324) aufweist und das elektrische Stimulationssystem (100; 800) ferner eine Leitung (103) aufweist, die mit dem Steuermodul (102) und der Leitungsverlängerung (324) koppelbar ist, wobei die Verbinderanordnung (322) Teil der Leitungsverlängerung (324) ist.

13. Elektrischer Kontakt (400; 500) für eine Leitungsanordnung, wobei der elektrische Kontakt (400; 500) aufweist:
einen Kontaktring (402); und
mehrere um den Kontaktring (402) verteilte Kugel-Feder-Anordnungen (408; 508), wobei jede Kugel-Feder-Anordnung (408; 508) aufweist: ein Gehäuse (410), das mit dem Kontaktring (402) gekoppelt ist, ein Vorspannteil (414; 514), das im Kugel-Feder-Gehäuse (410) angeordnet ist, und eine leitende Kugel (412), die mindestens teilweise im Kugel-Feder-Gehäuse (410) angeordnet ist, wobei das Kugel-Feder-Gehäuse (410) eine Öffnung definiert, die kleiner ist als ein Durchmesser der Kugel (412), und das Vorspannteil (414; 514) die Kugel (412) hin zur Öffnung drückt, so dass bei Fehlen einer dem Vorspannteil (414; 514) entgegenwirkenden Kraft sich ein Abschnitt der Kugel (412) aus der Öffnung im Kugel-Feder-Gehäuse (410) erstreckt.

14. Elektrischer Kontakt (400; 500) nach Anspruch 13, wobei die Kugel (412) im Kugel-Feder-Gehäuse (410) frei drehen kann.

15. Elektrischer Kontakt (400; 500) nach Anspruch 13 oder 14, wobei das Vorspannteil (414; 514) eine Spiral- oder Kegeldruckfeder ist.

## Revendications

1. Ensemble connecteur (144 ; 322) comprenant :
un boîtier de connecteur allongé (314) ayant une première extrémité, une deuxième extrémité opposée, et un axe longitudinal ;
un orifice (304 ; 330) défini au niveau de la première extrémité du boîtier de connecteur (314 ; 324), l'orifice (304 ; 330) étant configuré et agencé pour recevoir une extrémité proximale (306 ; 348) d'un fil ou d'une extension de fil (103 ; 324), dans lequel l'extrémité proximale (306 ; 348) du fil ou de l'extension de fil (103 ; 324) comprend une pluralité de bornes (310) électriquement isolées les unes des autres ;
une lumière définie dans le boîtier de connecteur (314 ; 324), la lumière s'étendant à partir de l'orifice (304 ; 330) le long de l'axe longitudinal du boîtier de connecteur (314 ; 324) ; et
une pluralité de contacts (316 ; 400 ; 500 ; 602) disposés dans le boîtier de connecteur allongé (314 ; 324) et électriquement isolés les uns des autres, au moins l'un de la pluralité de contacts (316 ; 400) étant configuré et agencé pour se coupler à au moins l'une de la pluralité de bornes (310) lorsque l'extrémité proximale du fil ou de l'extension de fil (103 ; 324) est reçue à l'intérieur de la lumière du boîtier de connecteur (314 ; 324) ;
dans lequel chacun de la pluralité de contacts (316 ; 400 ; 500) comprend un anneau de contact (402) et une pluralité d'ensembles de ressort à bille (408 ; 508) répartis autour de l'anneau de contact (402), chaque ensemble de ressort à bille (408 ; 508) comprenant un boîtier (410) couplé à l'anneau de contact (402), un élément de sollicitation (414 ; 514) disposé à l'intérieur du boîtier de ressort à bille (410), et une bille conductrice (412) disposée au moins partiellement à l'intérieur du boîtier de ressort à bille (410), dans lequel le boîtier de ressort à bille (410) définit une ouverture qui est inférieure à un diamètre de la bille (412) et l'élément de sollicitation (414 ; 514) pousse la bille (412) vers l'ouverture de sorte que, en l'absence d'une force qui s'oppose à l'élément de sollicitation (414 ; 514), une partie de la bille (412) s'étend hors de l'ouverture dans le boîtier de ressort à bille (410).

2. Ensemble connecteur (144 ; 322) selon la revendication 1, dans lequel le boîtier de ressort à bille (410) est un corps cylindrique.

3. Ensemble connecteur (144 ; 322) selon l'une quelconque des revendications 1 ou 2, comprenant en outre un congé (416) attenant au boîtier de ressort à bille (410) et une surface interne (404) de l'anneau de contact (402).

4. Ensemble connecteur (144 ; 322) selon l'une quelconque des revendications 1 à 3, dans lequel la bille (412) est libre de tourner à l'intérieur du boîtier de ressort à bille (410).

5. Ensemble connecteur (144 ; 322) selon l'une quelconque des revendications 1 à 4, dans lequel l'élément de sollicitation (414 ; 514) est un ressort de compression hélicoïdal ou conique.

6. Ensemble connecteur (144 ; 322) selon l'une quelconque des revendications 1 à 5, dans lequel la bille (412) est libre d'effectuer une translation à l'intérieur du boîtier (410).

7. Ensemble connecteur selon la revendication 6, dans lequel l'élément de sollicitation (414 ; 514) est configuré pour se déplacer dans une direction radiale par rapport à l'axe longitudinal du boîtier de connecteur allongé (314 ; 324).

8. Ensemble connecteur (144 ; 322) selon l'une quelconque des revendications 1 à 7, dans lequel l'élément de sollicitation (414 ; 514) est en outre disposé entre la bille (412) et une surface interne (404) de l'anneau de contact (402).

9. Ensemble de fil comprenant :
un fil ou une extension de fil (103 ; 324) ayant une extrémité proximale (306 ; 348) et une extrémité distale (326), dans lequel l'extrémité proximale (306 ; 348) du fil ou de l'extension de fil (103 ; 324) comprend une pluralité de bornes (310) électriquement isolées les unes des autres ; et
l'ensemble connecteur (144 ; 322) selon l'une quelconque des revendications 1 à 8.

10. Système de stimulation électrique (100 ; 800) comprenant :
l'ensemble de fil selon la revendication 9 ; et
un module de commande (102) couplé à l'ensemble de fil, le module de commande comprenant :
un boîtier (114), et
un sous-ensemble électronique (110 ; 810) disposé dans le boîtier (114).

11. Système de stimulation électrique (100 ; 800) selon la revendication 10, dans lequel l'ensemble connecteur (144 ; 322) fait partie du module de commande (102).

12. Système de stimulation électrique (100 ; 800) selon la revendication 10, dans lequel l'ensemble de fil comprend l'extension de fil (324) et le système de stimulation électrique (100 ; 800) comprend en outre un fil (103) pouvant être couplé au module de commande (102) et à l'extension de fil (324), dans lequel l'ensemble connecteur (322) fait partie de l'extension de fil (324).

13. Contact électrique (400 ; 500) pour un ensemble de fil, le contact électrique (400 ; 500) comprenant :
un anneau de contact (402) ; et
une pluralité d'ensembles de ressort à bille (408 ; 508) répartis autour de l'anneau de contact (402), chaque ensemble de ressort à bille (408 ; 508) comprenant un boîtier (410) couplé à l'anneau de contact (402), un élément de sollicitation (414 ; 514) disposé à l'intérieur du boîtier de ressort à bille (410), et une bille conductrice (412) disposée au moins partiellement à l'intérieur du boîtier de ressort à bille (410), dans lequel le boîtier de ressort à bille (410) définit une ouverture qui est inférieure à un diamètre de la bille (412) et l'élément de sollicitation (414 ; 514) pousse la bille (412) vers l'ouverture de sorte que, en l'absence d'une force qui s'oppose à l'élément de sollicitation (414 ;514), une partie de la bille (412) s'étend hors de l'ouverture dans le boîtier de ressort à bille (410).

14. Contact électrique (400 ; 500) selon la revendication 13, dans lequel la bille (412) est libre de tourner à l'intérieur du boîtier de ressort à bille (410).

15. Contact électrique (400 ; 500) selon l'une quelconque des revendications 13 ou 14, dans lequel l'élément de sollicitation (414 ; 514) est un ressort de compression hélicoïdal ou conique.
